# FASCICULE DE BREVET EUROPEEN

(11) **EP 3 400 060 B1**
(45) Date de publication et mention de la délivrance du brevet: **04.03.2020**
(21) Numéro de dépôt: 17700313.4
(22) Date de dépôt: 06.01.2017
(51) Int. Cl.: A61N 5/06, A61B 18/20, A61B 50/00, A61B 18/00

(54) **MANCHON DE PROTECTION POUR UN DISPOSITIF DE TRAITEMENT DERMATOLOGIQUE**
SCHUTZHÜLLE FÜR EINE DERMATOLOGISCHE BEHANDLUNGSVORRICHTUNG
PROTECTIVE SLEEVE FOR A DERMATOLOGICAL TREATMENT DEVICE

(30) Priorité: 07.01.2016 FR 1600034
(43) Date de publication de la demande: 14.11.2018
(73) Titulaire: Urgo Recherche Innovation et Développement, 21300 Chenôve (FR)
(72) Inventeur: LAMOISE, Michel, 21110 Bessey Les Cîteaux (FR)
(74) Mandataire: Barbot, Willy
(86) Numéro de dépôt international: PCT/EP2017/000012
(87) Numéro de publication internationale: WO 2017/118605

(56) Documents cités:
- WO-A1-99/52595
- US-A- 5 514 126

## Description

La présente invention concerne un dispositif de traitement dermatologique comprenant une tête laser apte à tirer un faisceau laser. Un tel dispositif est typiquement employé pour créer un échauffement d'une zone cible comprenant des tissus dermiques d'un patient afin d'en accélérer la cicatrisation (voir par exemple US 5514126).

Plus particulièrement, la présente invention concerne un manchon de protection apte à protéger le dispositif de traitement dermatologique pendant son utilisation, contre d'éventuelles souillures. En lien avec de telles souillures, le dispositif de traitement dermatologique devrait faire l'objet d'une forte stérilisation en vue de limiter les risques de transmission d'agents pathogènes. Maintenant, une telle stérilisation aurait entrainé des altérations du dispositif. Aussi, et pour limiter les risques sanitaires et la détérioration du dispositif, on utilise un manchon de protection qui est stérilisable ou, avantageusement, à usage unique. De la sorte, les risques de contamination sont limités et ceci sans compromettre la longévité du dispositif de traitement en lui même.

Le concept d'un tel manchon de protection est connu dans le domaine. En l'espèce, le manchon de l'invention comprend une coque rigide et une jupe souple ; la jupe souple étant assemblée sur le bord de la coque rigide. Ensemble, la coque rigide et la jupe souple forment une enveloppe entourant le dispositif de traitement dermatologique. La coque rigide est conformée de manière à pouvoir être mise en place sur la tête laser et comprend une fenêtre, apte à être disposée en regard du faisceau laser, afin de permettre au faisceau de traverser le manchon depuis le dispositif vers l'extérieur afin de permettre au faisceau de viser la zone cible de peau à traiter et ainsi réaliser le traitement. La coque rigide comprend encore une paroi d'appui disposée autour de la fenêtre. Cette paroi d'appui est apte, par sa surface externe, à venir en appui sur la peau du patient, autour de la zone cible, afin de permettre au faisceau de viser la zone cible. De plus, la paroi d'appui assure une préparation de la zone cible, en réalisant une mise en tension de la peau au niveau de la zone cible.

L'efficacité et la non-vulnérabilité du traitement nécessitent un positionnement précis et répétable, en distance et en orientation, du faisceau laser relativement à la zone cible. Ce positionnement peut être obtenu au moyen de la paroi d'appui, portée par le manchon, et d'un positionnement précis du manchon relativement à la tête laser et au faisceau laser.

Les manchons de l'art antérieur, n'assurent pas correctement cette fonction de positionnement précis, du fait principalement de moyens de fixation du manchon sur le dispositif de traitement absents ou insuffisamment efficaces à réaliser une immobilisation du manchon relativement au dispositif de traitement.

La présente invention propose un manchon amélioré qui évite cet inconvénient.

L'invention a pour objet un manchon de protection pour un dispositif de traitement dermatologique comprenant une tête laser apte à tirer un faisceau laser, le manchon comprenant une coque rigide conformée de manière à pouvoir être mise en place sur la tête laser et comprenant une fenêtre apte à être disposée en regard du faisceau laser, une paroi d'appui, disposée autour de la fenêtre, apte à venir en appui autour d'une zone cible, et un moyen de fixation avec le dispositif, où le moyen de fixation est disposé au plus près de la paroi d'appui.

Le moyen de fixation est disposé à moins de 10 mm de la paroi d'appui, de préférence à moins de 5 mm.

Selon une autre caractéristique, le moyen de fixation comprend au moins une protubérance, apte à venir se loger dans une cavité complémentaire du dispositif, respectivement une cavité apte à accueillir une protubérance complémentaire du dispositif, préférentiellement au moins trois protubérances et/ou cavités.

Selon une autre caractéristique, lesdites protubérances et/ou cavités sont angulairement équiréparties.

La fenêtre comprend encore une bordure, disposée sur toute la périphérie de la fenêtre, sensiblement perpendiculaire à la paroi d'appui et s'étendant vers l'intérieur du manchon.

Les inventeurs ont en effet constaté qu'une telle bordure permettait, en contribuant à rigidifier la coque malgré l'ouverture opérée au niveau de sa fenêtre, à maintenir la planéité de la paroi venant en appui autour de la zone cible et, de la sorte, le positionnement précis du la tête laser en regard de la zone cible.

De surcroît, la présence d'une telle bordure permet, alors que la paroi d'appui est appliquée avec force parfois, d'éviter de blesser le patient avec le bord de la fenêtre.

Selon une autre caractéristique, l'extension de la bordure est comprise entre 1 et 5 mm, préférentiellement elle est égale à 3 mm.

Selon une autre caractéristique, la fenêtre comprend encore une couronne, prolongeant la bordure, disposée sensiblement perpendiculaire à la bordure et s'étendant vers l'intérieur de la fenêtre.

Les inventeurs ont également constaté avec l'intégration d'une telle couronne en plus de la bordure, qu'outre l'obtention d'une rigidification encore améliorée de la coque, cette couronne permettait de mieux protéger la tête laser des souillures du patient. De ce fait, le dispositif de traitement dermatologique nécessitait une décontamination moins drastique entre deux traitements et présentait, en conséquence, une durée de vie améliorée.

Selon une autre caractéristique, l'extension de la couronne est comprise entre 0,5 et 4 mm, préférentiellement entre 2 et 2,2 mm.

Selon une autre caractéristique, la fenêtre est oblongue.

Les inventeurs ont en effet constaté qu'une telle forme, du fait de l'absence d'angles, limitait les blessures au patient et, simultanément, n'interférait pas avec le faisceau laser tout en conférant une bonne rigidité à l'ensemble.

Selon une autre caractéristique, la fenêtre présente un grand axe au moins égal à 23,75 mm et un petit axe au moins égal à 10,05 mm.

Selon une autre caractéristique, le manchon comprend encore une jupe souple solidaire de la coque rigide et conformée de telle manière à former avec la coque rigide une enveloppe entourant le dispositif, où la jupe souple comprend au moins une ouverture de ventilation.

D'autres caractéristiques, détails et avantages de l'invention ressortiront plus clairement de la description détaillée donnée ci-après à titre indicatif en relation avec des dessins sur lesquels :
- les figures 1-4 présentent un mode de réalisation préférentiel d'une coque rigide d'un manchon de protection,
- la figure 1 présente une vue en perspective de la coque rigide,
- la figure 2 présente une vue de face de cette même coque,
- la figure 3 présente une vue de dessous de la coque rigide coupée selon AA,
- la figure 4 présente une vue de côté de la coque rigide coupée selon BB.
- la figure 5 présente une vue de côté de l'ensemble manchon avec sa coque rigide et sa jupe souple.
- la figure 6 présente une vue de dessus de ce même ensemble manchon.

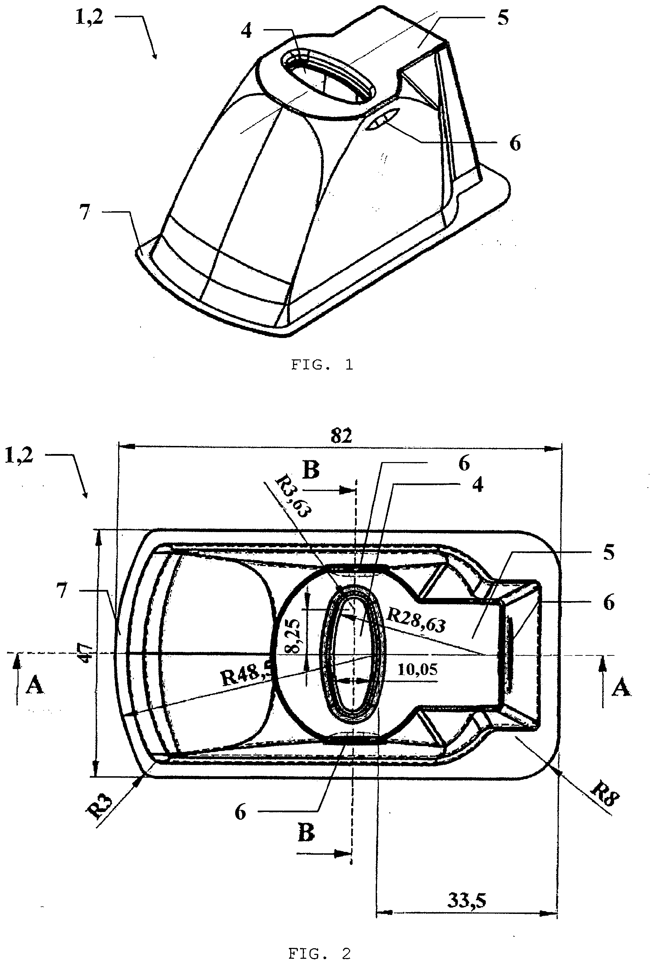

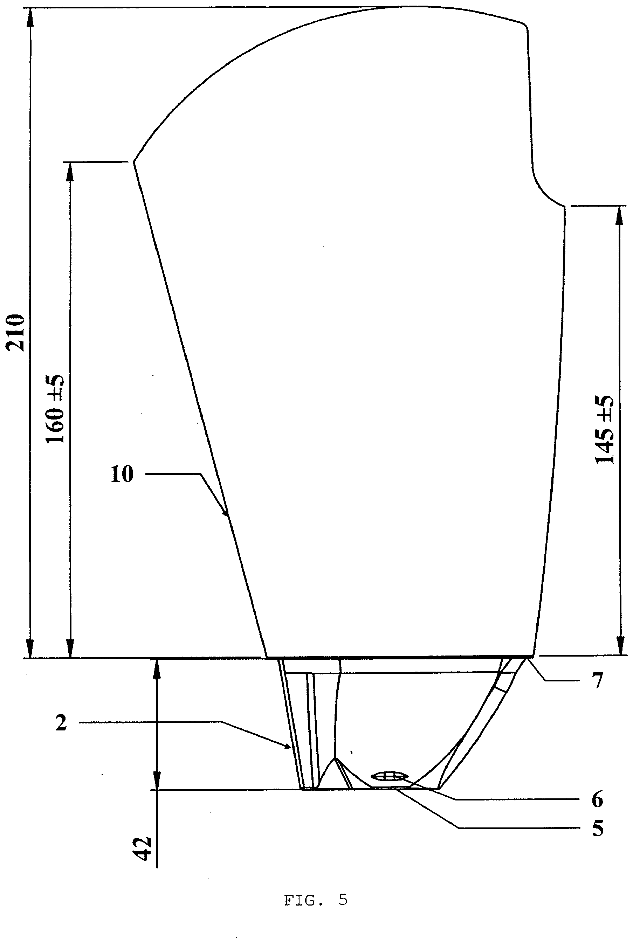

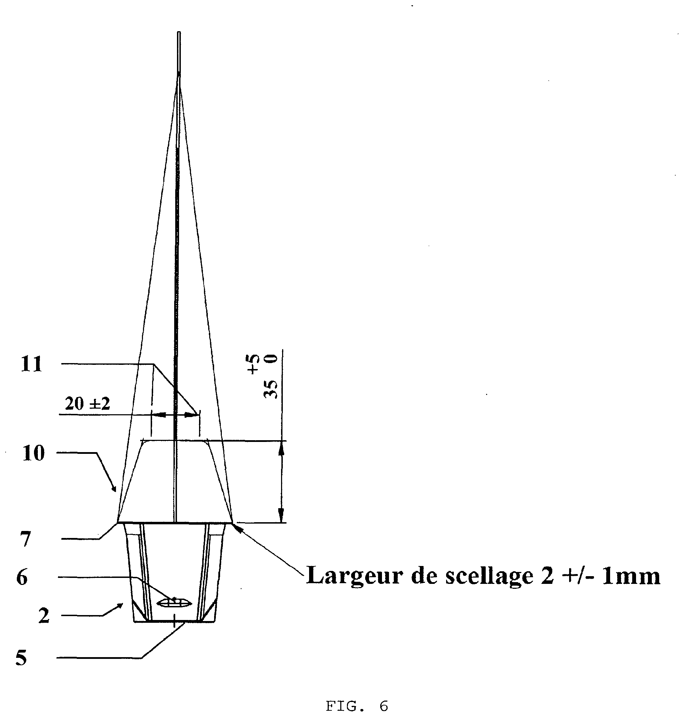

Les figures 1-4 montrent une coque rigide 2 d'un manchon de protection 1 pour un dispositif de traitement dermatologique. Les figures 5-6 montrent elles un manchon de protection 1 avec sa coque rigide 2 et sa jupe souple 10. Le dispositif de traitement dermatologique (non représenté) comprend une tête laser apte à tirer un faisceau laser. Un exemple d'un tel dispositif de traitement dermatologique est donné par le brevet européen EP 2519176. La coque rigide 2 est apte à venir recouvrir la tête laser. La coque rigide est avantageusement réalisée en matériau plastique rigide, tel un PVC rigide, un PMMA (polyméthylméthacrylate), un polystyrène ou encore un polymère fluoré. Ceci permet de réaliser les fonctions attendues pour un coût faible permettant que la coque rigide 2 reste jetable. Le matériau de la coque peut également contenir un ou plusieurs filtres de protection évitant la réflexion du rayonnement laser. La coque est de préférence transparente ou translucide pour faciliter, d'une part, la mise en place du manchon sur le dispositif de traitement dermatologique et, d'autre part, l'utilisation dudit dispositif. La coque est préférentiellement sans ouvertures, cela permet d'éviter la réflexion du rayon laser vers l'utilisateur ou le patient. Cela permet également d'éviter de souiller le dispositif de traitement. La jupe souple 10 vient recouvrir le reste du dispositif de traitement et est réalisée en film plastique mince et, de préférence, transparent ou translucide. A titre d'exemple, celle-ci peut être réalisée en polyuréthane ou en PVC souple. Pour former le manchon de protection 1, la jupe souple 10 et la coque rigide 2 sont scellées par des techniques bien connues, par collage ou soudage à chaud par exemple.

Le manchon 1 ne doit pas faire obstacle au faisceau laser. Aussi, le manchon 1 comprend une fenêtre 4 découpée dans la coque rigide 2. Cette fenêtre 4 est conformée de manière à être disposée en regard du faisceau laser, avantageusement de manière centrée et perpendiculaire relativement au faisceau laser, lorsque la coque rigide 2 est mise en place sur la tête laser du dispositif. La fenêtre 4 est une découpe dans la coque rigide 2 et ne contient aucune matière, afin de ne pas risquer d'atténuer, de réfléchir ou de dévier le faisceau laser.

La coque rigide 2 comprend encore une paroi d'appui 5. Cette paroi d'appui 5 est disposée autour de la fenêtre 4, et est apte à venir en appui autour de la zone cible. Cette paroi d'appui 5 présente une forme quelconque, de préférence adaptée au dispositif, par exemple plane, comme dans le mode de réalisation représenté. Cette paroi d'appui 5 a au moins deux fonctions. Une première fonction est une fonction de préparation de la zone cible, par exemple en réalisant un lissage ou une mise en tension de la peau. Une deuxième fonction est un positionnement, tant en orientation qu'en distance du faisceau laser relativement à la zone cible. Pour cela la paroi d'appui 5 est avantageusement sensiblement perpendiculaire au faisceau laser, une fois la coque rigide 2 immobilisée relativement à la tête laser et au faisceau laser.

Afin de réaliser cette immobilisation efficacement, la coque rigide 2 comprend encore un moyen de fixation 6, permettant une fixation de la coque rigide 2 avec le dispositif de traitement, par exemple au niveau de la tête laser.

Certains manchons de l'art antérieur ne comportent pas de moyen de fixation et réalisent un positionnement du faisceau laser relativement à la zone cible au moyen d'un appui d'une face d'appui présente sur la tête laser sur la surface interne de la paroi d'appui de la coque rigide. Un tel empilage n'est pas efficace à garantir une distance constante à la zone cible et encore moins une orientation constante.

D'autres manchons de l'art antérieur comportent un moyen de fixation, situé à proximité du bord 7 de la coque rigide 2 du manchon 1. Une telle localisation du moyen de fixation autorise une déformation de la coque rigide entre le moyen de fixation et la paroi d'appui. Aussi une orientation constante et/ou une distance constante du faisceau laser relativement à la zone cible n'est pas garantie.

Selon une caractéristique importante de l'invention, le moyen de fixation 6 est disposé très près de la paroi d'appui 5. Ainsi dans le mode de réalisation illustré aux figures 1-6, le moyen de fixation 6, qui comprend trois clips, est disposé sur les parois en retour de la paroi d'appui 5, au plus près de la paroi d'appui 5, soit aussi près de l'angle entre la paroi en retour et la paroi d'appui 5 que le permettent les contraintes de réalisation du manchon 1. En plus des moyens de fixation situés près de la paroi d'appui (moyens proximaux), on peut envisager en outre des moyens de fixation à distance de la paroi d'appui (moyens distaux). De façon générale, les moyens de fixation peuvent être concaves ou convexes.

Ainsi disposé, le moyen de fixation 6 garantit une immobilisation de la coque rigide 2 et du manchon 1 en général relativement à la tête laser, de sorte que la distance entre le moyen de fixation 6 et la paroi d'appui 5, qui pourrait éventuellement permettre une déformation, est extrêmement réduite. Ceci garantit un positionnement idéal du faisceau laser relativement à la paroi d'appui 5 et donc relativement à la zone cible.

Avantageusement le moyen de fixation 6 est tel qu'il garantisse un positionnement unique du manchon 1 relativement au dispositif, assurant ainsi une répétabilité du procédé de traitement. La coque rigide 2 est le plus souvent conformée pour reproduire sensiblement la forme de la tête laser qu'elle recouvre, afin que la coque rigide 2 épouse la tête laser.

Dans le mode de réalisation illustré, la disposition des moyens de fixation 6, ne permet qu'une seule position de la coque rigide 2 sur la tête laser. La forme non révolutive de la tête laser et de la coque rigide 2 assure un détrompage en rotation. Ainsi il existe une unique position dans laquelle la coque rigide 2 peut venir se positionner et être fixée sur la tête laser du dispositif de traitement. Ceci garantit aussi que la fenêtre 4 est bien positionnée et orientée en regard du faisceau laser.

Selon un mode de réalisation, tel qu'illustré, le moyen de fixation 6 comprend au moins une protubérance 6. Cette protubérance 6 est conformée et dimensionnée de manière à pouvoir venir se loger dans une cavité (non représentée) complémentaire réalisée dans le dispositif, typiquement au niveau de la tête laser, et disposée en regard. De préférence, la protubérance est de forme ovoïde. Lors de la mise en place de la coque rigide 2 sur le dispositif, l'élasticité de la coque rigide 2 peut être mise à profit afin d'écarter légèrement la protubérance 6 lors de l'approche de la cavité, pour ensuite la laisser revenir en position de repos, lorsque la protubérance 6 s'engage dans la cavité. Cette élasticité assure alors un maintien de la protubérance 6 dans la cavité. La protubérance 6 permet, en coopérant avec la cavité, de réaliser un clipsage assurant la fixation de la coque rigide 2 sur le dispositif. Ce clipsage est réversible et permet de retirer le manchon 1 après usage. Un manchon neuf peut être installé à chaque nouveau patient.

De manière alternative, mais équivalente, le moyen de fixation peut comprendre une cavité (non représentée) apte à accueillir une protubérance complémentaire du dispositif.

La coque peut également présenter des moyens de guidage, sous forme de rainure par exemple, afin de faciliter le positionnement du manchon sur le dispositif de traitement.

Il peut également être envisagé des moyens facilitant le retrait du manchon. Ces moyens peuvent se présenter sous forme d'un renfoncement ou d'une bordure au niveau de la coque.

Ces modes de réalisation peuvent être combinés, en panachant les cavités et/ou les protubérances.

Une seule paire cavité/protubérance peut suffire à réaliser un moyen de fixation 6. Cependant, le nombre de paires cavité/protubérance peut être augmenté. Un nombre préférentiel de trois paires de protubérances et/ou cavités est avantageux, en ce qu'il garantit un positionnement mécaniquement précis et répétable de la coque rigide 2 relativement au dispositif. Un nombre supérieur, bien que quasi-statique, permet d'augmenter la tenue ou la rigidité de fixation.

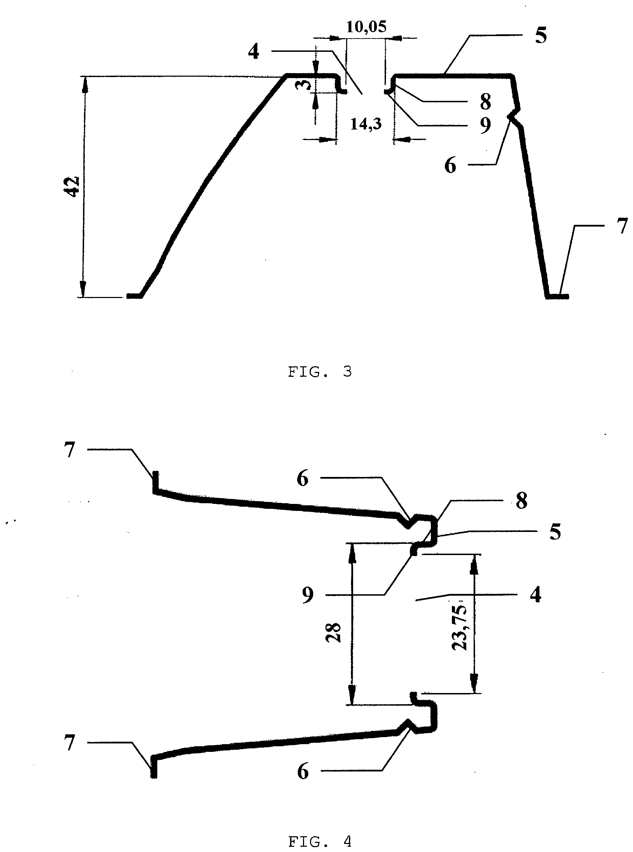

Dans le mode de réalisation illustré aux figures 1-6, trois protubérances 6 sont réalisées dans la coque rigide 2. Une première protubérance 6, plus particulièrement visible sur la figure 3, est disposée sur la face latérale droite de la coque rigide 2. Deux autres protubérances 6 opposées, sensiblement parallèles entre elles et perpendiculaires à la précédente sont disposées respectivement sur la face supérieure et sur la face inférieure. Elles sont plus particulièrement visibles aux figures 1 et 4. Les trois protubérances 6 sont simultanément visibles à la figure 2.

Selon un mode de réalisation avantageux, afin de répartir les contraintes et assurer le positionnement relatif de la coque rigide 2 relativement au dispositif, lesdites protubérances 6 et/ou cavités sont avantageusement sensiblement angulairement équiréparties.

Un autre problème occasionné par les manchons selon l'art antérieur apparait au niveau de la fenêtre 4. La fenêtre doit laisser passer les différents faisceaux, dont le faisceau laser, sans risque de le dévier, de l'atténuer ou de le réfléchir. Aussi la fenêtre 4 est une découpe pratiquée dans la coque rigide 2. La fenêtre 4 constitue ainsi une ouverture au niveau de laquelle le dispositif n'est pas protégé et par laquelle d'éventuelles souillures issues du patient, telles que du sang, pouvant être contaminantes, peuvent, au cours du traitement, transiter pour atteindre le dispositif.

Relativement à l'art antérieur où la fenêtre est une simple découpe dans la paroi d'appui, l'invention corrige cet inconvénient. Selon une caractéristique de l'invention, la fenêtre 4 de la coque 2 voit son profil modifié afin de réduire ou d'éliminer ce risque de contamination.

Pour cela, la fenêtre 4 comprend encore une bordure 8, disposée sur toute la périphérie de la fenêtre 4, sensiblement perpendiculaire à la paroi d'appui 5 et s'étendant vers l'intérieur du manchon 1. Une telle bordure 8 est plus particulièrement visible aux figures 3 et 4. Cette bordure 8 est ainsi rentrante vers l'intérieur du manchon 1 et vers le dispositif afin de mieux le protéger contre d'éventuelles souillures, le cas échéant en rentrant dans une cavité de la tête laser. Une telle bordure 8 peut être utilisée seule, sans la couronne 9.

Une bordure 8 présente une extension sensiblement constante le long du périmètre de la fenêtre 4. Cette extension est typiquement comprise entre 1 et 5 mm. Elle est préférentiellement égale à 3 mm.

Avantageusement, afin d'améliorer encore la protection du dispositif contre les souillures, la fenêtre 4 comprend encore une couronne 9. Cette couronne 9 prolonge la bordure 8 précédente. La couronne 9 est disposée sensiblement perpendiculaire à la bordure 8 et s'étend vers l'intérieur/vers le centre de la fenêtre 4. Une telle couronne 9 est plus particulièrement visible aux figures 3 et 4. Il résulte des constructions précédentes que la couronne 9 est sensiblement parallèle à la paroi d'appui 5. Ceci permet de former un écran parallèle et distant de la zone cible. La distance permet d'éviter des souillures par contact. La couronne 9, ainsi éloignée de la paroi d'appui 5 par la bordure 8, permet d'éloigner la fenêtre 4 de la zone cible, potentiellement contaminante. La paroi de la couronne 9 assure cependant une protection contre d'éventuelles projections.

Une couronne 9 présente une extension typiquement comprise entre 0,5 et 4 mm. Cette extension est préférentiellement entre 2 et 2,2 mm.

Il va de soi que la dimension de la fenêtre 4, en tant que partie découpée dans la coque rigide 2, reste identique, en ce qu'elle est déterminée par les contraintes liées au passage du faisceau laser. Ainsi la couronne 9 ne vient pas en déduction de la fenêtre 4. Au contraire la présence de la couronne 9 nécessite un décrochement dans la paroi d'appui 5, augmenté de la taille de la couronne 9.

Le faisceau laser employé présente une forme sensiblement rectangulaire. Il ne faut en aucun cas que le faisceau laser interfère avec l'un des contours de la fenêtre 4. Aussi, il convient que la fenêtre 4 présente une découpe englobant au moins cette forme rectangulaire. La coque rigide 2 est typiquement réalisée par thermoformage pour le corps, avec une reprise ultérieure de découpe pour la réalisation de la fenêtre 4. La tolérance de positionnement lors de l'opération de découpe nécessite d'augmenter la taille de la fenêtre 4.

Selon un mode de réalisation évident, employé dans l'art antérieur, la fenêtre est dimensionnée selon un rectangle reproduisant la forme rectangulaire du faisceau laser, homothétiquement augmenté sur toutes ses dimensions, de la tolérance de l'opération de découpe, conduisant à un rectangle de plus grandes dimensions.

Une telle forme conduit cependant à retirer beaucoup de matière à la paroi d'appui 5 ce qui peut la fragiliser. De plus les coins de ladite forme rectangulaire créent des amorces de ruptures, augmentant encore la fragilisation.

Aussi, selon une caractéristique de l'invention, la forme de la fenêtre 4 est oblongue.

Selon un mode de réalisation, cet oblong est déterminé de manière à satisfaire deux contraintes. D'une part la forme oblongue est telle qu'elle circonscrive le rectangle du faisceau laser. D'autre part, l'oblong est tel que ses dimensions selon le petit et le grand axe soient égales aux dimensions du faisceau laser augmentées de la tolérance de l'opération de découpe. Il en résulte ainsi que la forme oblongue permet d'accommoder la forme rectangulaire du faisceau laser tout en autorisant un décalage de la fenêtre 4 causée par un désalignement lors de l'opération de découpe, au moins le long des axes de l'oblong. Ceci permet avantageusement de réaliser une fenêtre 4 moins échancrée, en ce qu'elle ne comprend pas les coins découpés d'un rectangle. Une telle fenêtre 4 confère une meilleure résistance à la paroi d'appui 5.Cette forme oblongue avec des angles arrondis et sans découpes directes dans le plan avec la peau du patient évite toutes coupures blessantes pour le patient.

A titre indicatif dans le mode de réalisation illustré le faisceau laser présente une dimension de 20 x 4 mm. La tolérance de l'opération de découpe est typiquement de +/-1,5 mm, se décomposant en +/-1 mm pour l'opération proprement dite et +/-0,5 mm de tolérance générale sur les côtes hors tout. Ceci conduit selon l'art antérieur à une fenêtre rectangulaire de 27 x 9 mm. Au contraire, selon l'invention, une fenêtre 4 oblongue présente un grand axe au moins égal à 23,25 mm et un petit axe au moins égal à 10,05 mm.

Le manchon 1 comprend typiquement une coque rigide 2 pour les fonctions nécessitant de la rigidité, telles que celle vue précédemment de positionnement, en orientation et distance, du faisceau laser relativement à la zone cible.

Cette coque rigide 2 permet d'entourer partiellement une partie portative et mobile du dispositif, appelée pièce à main. Le manchon 1 comprend encore une jupe souple 10, solidaire de la coque rigide 2, typiquement soudée au niveau du bord 7. Cette jupe souple 7 est typiquement réalisée en PVC souple.

Cette jupe souple 10 est conformée de manière à compléter, avec la coque rigide 2, une enveloppe entourant le dispositif. Cette enveloppe entoure le dispositif et le manchon 1 nécessite au moins une ouverture de ventilation 11. L'ouverture nécessaire que constitue la fenêtre 4 n'est pas suffisante à ventiler efficacement. Selon l'art antérieur, une telle ouverture de ventilation 11 est ménagée dans la coque rigide. Ceci est préjudiciable en ce qu'une telle ouverture affecte la résistance mécanique de la coque rigide.

Aussi, selon une autre caractéristique de l'invention, la ou les ouvertures de ventilation 11 sont avantageusement ménagées dans la jupe souple. Ceci présente l'avantage de pouvoir supprimer les ouvertures de ventilation pratiquées dans la coque rigide 2, augmentant ainsi sa résistance. Un autre avantage est de faciliter l'opération de réalisation des ouvertures de ventilation, le matériau de la jupe souple étant plus facile à découper.

Le manchon est de préférence conditionné avec la jupe retroussée afin de faciliter sa mise en place sur le dispositif de traitement au moment de l'utilisation.

Préférentiellement, le manchon est emballé dans un étui et stérilisé. L'invention est définie dans les revendications, d'autres modes de réalisation étant exemplaires.

## Revendications

1. Un manchon de protection (1) pour un dispositif de traitement dermatologique comprenant une tête laser apte à tirer un faisceau laser, le manchon (1) comprenant une coque rigide (2) conformée de manière à pouvoir être mise en place sur la tête laser et comprenant une fenêtre (4) apte à être disposée en regard du faisceau laser, une paroi d'appui (5), disposée autour de la fenêtre (4), apte à venir en appui autour d'une zone cible, et un moyen de fixation avec le dispositif ***caractérisé en ce que*** le moyen de fixation est disposé à moins de 10 mm, de préférence à moins de 5 mm, de la paroi d'appui (5)et la fenêtre (4) comprend encore une bordure (8), disposée sur toute la périphérie de la fenêtre (4), sensiblement perpendiculaire à la paroi d'appui (5) et s'étendant vers l'intérieur du manchon (1).

2. Le manchon (1) selon la revendication **1**, où le moyen de fixation comprend au moins une protubérance (6), apte à venir se loger dans une cavité complémentaire du dispositif, respectivement une cavité apte à accueillir une protubérance complémentaire du dispositif, préférentiellement au moins trois protubérances et/ou cavités.

3. Le manchon (1) selon la revendication **2,** où lesdites protubérances (6) et/ou cavités sont angulairement équiréparties.

4. Le manchon (1) selon la revendication **1,** où l'extension de la bordure (8) est comprise entre 1 et 5 mm, préférentiellement égale 3 mm.

5. Le manchon (1) selon l'une quelconque des revendications 1 ou **4**, où la fenêtre (4) comprend encore une couronne (9), prolongeant la bordure (8), disposée sensiblement perpendiculaire à la bordure (8) et s'étendant vers l'intérieur de la fenêtre (4).

6. Le manchon (1) selon la revendication **5**, où l'extension de la couronne (9) est comprise entre 0,5 et 4 mm, préférentiellement entre 2 et 2,2 mm.

7. Le manchon (1) selon l'une quelconque des revendications **1** à **6**, où la fenêtre (4) est oblongue.

8. Le manchon (1) selon la revendication **7**, où la fenêtre (4) présente un grand axe au moins égal à 23,75 mm et un petit axe au moins égal à 10,05 mm.

9. Le manchon (1) selon l'une quelconque des revendications **1** à **8**, comprenant encore une jupe souple solidaire de la coque rigide (2) et conformée de telle manière à former avec la coque rigide (2) une enveloppe entourant le dispositif, *c**aractérisé en ce que*** la jupe souple (10) comprend au moins une ouverture de ventilation (11).

## Patentansprüche

1. Schutzhülse (1) für eine dermatologische Behandlungsvorrichtung, die einen Laserkopf umfasst, der geeignet ist, einen Laserstrahl auszusenden, wobei die Hülse (1) eine starre Schale (2) aufweist, dafür ausgebildet, auf den Laserkopf gesetzt zu werden, und ein Fenster (4) umfassend, das geeignet ist, dem Laserstrahl gegenüber angeordnet zu werden, eine um das Fenster (4) angeordnete Anlagewand (5), geeignet, um einen Zielbereich herum angelegt zu werden, und ein Mittel zur Befestigung an der Vorrichtung, ***dadurch gekennzeichnet, dass*** das Befestigungsmittel in weniger als 10 mm, vorzugsweise weniger als 5 mm von der Anlagewand (5) angeordnet ist und das Fenster (4) außerdem einen Rand (8) aufweist, der auf der gesamten Umfangslinie des Fensters (4) angeordnet ist, im Wesentlichen senkrecht zur Anlagewand (5) und ins Innere der Hülse (1) ragend.

2. Hülse (1) nach Patentanspruch 1, in der das Befestigungsmittel mindestens eine Vorsprung (6) umfasst, der geeignet ist, sich in einen komplementären Hohlraum der Vorrichtung einzufügen, bzw. einen Hohlraum, der geeignet ist, einen komplementären Vorsprung der Vorrichtung aufzunehmen, vorzugsweise mindestens drei Vorsprünge und/oder Hohlräume.

3. Hülse (1) nach Patentanspruch 2, in der die genannten Vorsprünge (6) und/oder Hohlräume mit gleichen Winkeln untereinander verteilt sind.

4. Hülse (1) nach Patentanspruch 1, in der die Ausdehnung des Randes (8) zwischen 1 und 5 mm beträgt, vorzugsweise 3 mm.

5. Hülse (1) nach irgendeinem der Patentansprüche 1 oder 4, in der das Fenster (4) außerdem einen Kranz (9) aufweist, der den Rand (8) fortsetzt, der im Wesentlichen senkrecht zum Rand (8) angeordnet ist und ins Innere des Fensters (4) ragt.

6. Hülse (1) nach Patentanspruch 5, in der die Ausdehnung des Kranzes (9) zwischen 0,5 und 4 mm beträgt, vorzugsweise zwischen 2 und 2,2 mm.

7. Hülse (1) nach irgendeinem der Patentansprüche 1 bis 6, in der das Fenster (4) länglich ist.

8. Hülse (1) nach Patentanspruch 7, in der das Fenster (4) eine große Achse von mindestens 23,75 mm und eine kleine Achse von mindestens 10,05 mm aufweist.

9. Hülse (1) nach irgendeinem der Patentansprüche 1 bis 8, außerdem eine verformbare Schürze mit der starren Schale (2) verbunden aufweisend und derart ausgebildet, dass sie mit der starren Schale (2) eine Hülle bildet, die die Vorrichtung umgibt, ***dadurch gekennzeichnet, dass*** die verformbare Schürze (10) mindestens eine Lüftungsöffnung (11) aufweist.

## Claims

1. A protective sleeve (1) for a dermatological treatment device comprising a laser head able to fire a laser beam, the sleeve (1) comprising a rigid shell (2) configured so as to be able to be placed on the laser head and comprising a window (4) able to be arranged across from the laser beam, a contact wall (5), arranged around the window (4), able to come into contact around a target zone, and an attachement mean to said device, ***characterized in that*** the attachment mean is positioned less than 10 mm, preferably less than 5 mm, from the contact wall (5) and the window (4) further comprises a border (8), arranged over the entire periphery of the window (4), substantially perpendicular to the contact wall (5) and extending toward the inside of the sleeve (1).

2. The sleeve (1) according to claim **1**, where the attachment means comprises at least one protuberance (6), able to be housed in a complementary cavity of the device, respectively a cavity able to accommodate a complementary protuberance of the device, preferably at least three protuberances and/or cavities.

3. The sleeve (1) according to claim **2**, where said protuberances (6) and/or cavities are angularly evenly distributed.

4. The sleeve (1) according to claim **1**, where the extension of the border (8) is comprised between 1 and 5 mm, preferably equal to 3 mm.

5. The sleeve (1) according to any one of claims **1** or **4**, where the window (4) further comprises a ring (9), extending the border (8), positioned substantially perpendicular to the border (8) and extending toward the inside of the window (4).

6. The sleeve (1) according to claim **5**, where the extension of the ring (9) is comprised between 0,5 and 4 mm, preferably between 2 and 2,2 mm.

7. The sleeve (1) according to any one of claims **1** to **6**, where the window (4) is oblong.

8. The sleeve (1) according to claim **7**, where the window (4) has a large axis at least equal to 23,75 mm and a small axis at least equal to 10,05 mm.

9. The sleeve (1) according to any one of claims **1** to **8**, further comprising a flexible skirt secured to the rigid shell (2) and configured so as to form, with the rigid shell (2), an enclosure surrounding the device, ***characterized in that*** the flexible skirt (10) comprises at least one ventilation opening (11).
